(19) Europäisches Patentamt
European Patent Office
Office européen des brevets

(11) **EP 3 914 628 B1**

(12) **EUROPÄISCHE PATENTSCHRIFT**

(45) Veröffentlichungstag und Bekanntmachung des Hinweises auf die Patenterteilung:
**11.03.2026   Patentblatt 2026/11**

(21) Anmeldenummer: **20700287.4**

(22) Anmeldetag: **13.01.2020**

(51) Internationale Patentklassifikation (IPC):
*C08F 6/00* (2006.01)      *C08F 4/28* (2006.01)
*C08F 4/40* (2006.01)      *C08J 3/24* (2006.01)
*C08F 2/24* (2006.01)      *C08F 2/10* (2006.01)
*C08F 120/06* (2006.01)    *C08F 220/06* (2006.01)

(52) Gemeinsame Patentklassifikation (CPC):
(C-Sets verfügbar)
**C08F 2/10; C08F 4/28; C08F 4/40; C08F 6/008; C08F 120/06; C08F 220/06; C08J 3/245;**
C08J 2333/02                                (Forts.)

(86) Internationale Anmeldenummer:
**PCT/EP2020/050627**

(87) Internationale Veröffentlichungsnummer:
**WO 2020/151970 (30.07.2020 Gazette 2020/31)**

(54) **VERFAHREN ZUR HERSTELLUNG VON SUPERABSORBERPARTIKELN**

METHOD FOR PRODUCING SUPERABSORBENT PARTICLES

PROCÉDÉ DE PRODUCTION DE PARTICULES SUPERABSORBANTES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.01.2019   EP 19153439**

(43) Veröffentlichungstag der Anmeldung:
**01.12.2021   Patentblatt 2021/48**

(73) Patentinhaber: **BASF SE**
**67056 Ludwigshafen am Rhein (DE)**

(72) Erfinder:
• **HAAG, Monica**
  **67056 Ludwigshafen (DE)**
• **GIERESCHER, Stefan**
  **67056 Ludwigshafen (DE)**

(74) Vertreter: **BASF IP Association**
**BASF SE**
**GBI - Z078**
**67056 Ludwigshafen (DE)**

(56) Entgegenhaltungen:
EP-A1- 3 000 486          WO-A1-2016/134905
DE-A1- 102005 011 165     DE-A1- 102017 205 365
US-A1- 2019 135 993

(52) Gemeinsame Patentklassifikation (CPC): (Forts.)

C-Sets
**C08F 6/008, C08L 33/02;**
**C08F 220/06, C08F 222/103**

## Beschreibung

Die vorliegende Erfindung betrifft ein Verfahren zur Herstellung von Superabsorberpartikeln, umfassend Polymerisation einer Monomerzubereitung, Trocknung des erhaltenen wässrigen Polymergels, Mahlung, Klassierung und thermische Oberflächennachvernetzung, wobei die Monomerzubereitung durch Mischen einer wässrigen Monomerlösung und einer geschäumten wässrigen Tensidlösung hergestellt wird.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Zur Verbesserung der Anwendungseigenschaften, wie beispielsweise Permeabilität (SFC) und Absorption unter einem Druck von 49,2 $g/cm^2$ (AUHL), werden Superabsorberpartikel im allgemeinen oberflächennachvernetzt. Dadurch steigt der Vernetzungsgrad der Partikeloberfläche, wodurch die Absorption unter einem Druck von 49,2 $g/cm^2$ (AUHL) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der Polymerpartikel kovalente Bindungen bilden können.

EP 2 518 092 A1 beschreibt die Herstellung poröser Superabsorberpartikel durch Aufschäumen der Monomerlösung vor der Polymerisation.

DE10 2005 011165 A1 beschreibt die Herstellung von superabsorbierendem Polymerschaum enthaltend Holzzellstoff oder Altpapier.

Aufgabe der vorliegenden Erfindung war die Bereitstellung verbesserten Verfahrens zur Herstellung von Superabsorberpartikeln, insbesondere für Superabsorberpartikel mit einer höheren Flüssigkeitsaufnahme.

Gelöst wurde die Aufgabe durch ein Verfahren zur Herstellung von Superabsorberpartikeln, umfassend Polymerisation einer Monomerzubereitung, Trocknung des erhaltenen wässrigen Polymergels, Mahlung, Klassierung und thermische Oberflächennachvernetzung, dadurch gekennzeichnet, dass die Monomerzubereitung durch Mischen einer wässrigen Monomerlösung, enthaltend

    a) mindestens ein ethylenisch ungesättigtes, säuregruppentragendes Monomer, das zumindest teilweise neutralisiert ist,
    b) mindestens einen Vernetzer und
    c) mindestens einen Initiator,

und einer geschäumten wässrigen Tensidlösung hergestellt wird.

In einer bevorzugten Ausführungsform der vorliegenden Erfindung wird die Polymerisation der Monomerzubereitung in einem Knetreaktor durchgeführt. Die wässrige Monomerlösung und die geschäumte wässrige Tensidlösung werden dabei im Knetreaktor gemischt. Der Knetreaktor kann kontinuierlich oder diskontinuierlich betrieben werden. Ein kontinuierlicher Knetreaktor ist bevorzugt.

Als Initiatoren c) sind Redox-Initiatoren besonders geeignet. Ein Redox-Initiator besteht aus einer oxidierenden Komponente, beispielsweise Natriumperoxodisulfat und/oder Wasserstoffperoxid, und einer reduzierenden Komponente, beispielsweise Ascorbinsäure. Die reduzierende Komponente wird vorzugsweise erst im Knetreaktor zugesetzt.

Die wässrige Monomerlösung enthält vorzugsweise von 30 bis 60 Gew.-%, besonders bevorzugt von 35 bis 65 Gew.-%, ganz besonders bevorzugt von 40 bis 50 Gew.-%, des Monomeren a). Das Monomer a) ist vorzugsweise von 40 bis 90 mol-%, besonders bevorzugt von 50 bis 85 mol-%, ganz besonders bevorzugt von 60 bis 80 mol-%, neutralisiert. Das bevorzugte Monomer a) ist teilneutralisierte Acrylsäure.

Die geschäumte wässrige Tensidlösung enthält vorzugsweise von 0,1 bis 10 Gew.-%, besonders bevorzugt von 0,5 bis 5 Gew.-%, ganz besonders bevorzugt von 1 bis 3 Gew.-%, mindestens eines Tensids. Die bevorzugten Tenside sind nichtionische Tenside, beispielsweise ethoxilierte $C_{14}$-$C_{20}$-Alkohole.

Die Tensidlösung wird erst geschäumt und erst die bereits geschäumte Tensidlösung wird mit der Monomerlösung gemischt.

Das Gewichtsverhältnis von geschäumter wässriger Tensidlösung zu wässriger Monomerlösung in der Monomerzubereitung beträgt vorzugsweise von 0,01 bis 0,30, besonders bevorzugt von 0,02 bis 0,20, ganz besonders bevorzugt von 0,03 bis 0,10.

Der vorliegenden Erfindung liegt die Erkenntnis zugrunde, dass die Reihenfolge der Verfahrensschritte einen entscheidenden Einfluss auf die Eigenschaften der erhaltenen Superabsorberpartikel hat. Die erfindungsgemäße Reihenfolge der Verfahrensschritte, d.h. das Mischen einer bereits geschäumten Tensidlösung mit einer Monomerlösung, führt zu einer höheren Maximaltemperatur bei der Polymerisation. Die Zentrifugenretentionskapazität (CRC) und die Extrahierbaren des Grundpolymers sind erhöht. Nach der thermischen Oberflächennachvernetzung weisen die gemäß dem erfindungsgemäßen Verfahren hergestellten Superabsorberpartikel eine deutlich verbesserte volumetrische Flüssigkeitsaufnahme unter 0,3 psi (2,07 kPa) Druck (VAUL), eine leicht verbesserte Absorption unter einem Druck von 49,2 g/cm$^2$ (AUHL) und eine vergleichbare Zentrifugenretentionskapazität (CRC) auf.

Die geschäumte wässrige Tensidlösung kann zusätzlich ein wasserlösliches Polymer enthalten, vorzugsweise von 0,5 bis 20 Gew.-%, besonders bevorzugt von 2 bis 15 Gew.-%, ganz besonders bevorzugt von 5 bis 10 Gew.-%. Die bevorzugten wasserlöslichen Polymere sind Polyethylenglykole.

Im Folgenden wird die Herstellung der Superabsorberpartikel näher erläutert:

Die Superabsorberpartikel werden durch Polymerisation einer Monomerlösung hergestellt und sind üblicherweise wasserunlöslich.

Die Monomeren a) sind vorzugsweise wasserlöslich, d.h. die Löslichkeit in Wasser bei 23° C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Die Monomere a) enthalten üblicherweise Polymerisationsinhibitoren, vorzugsweise Hydrochinonhalbether, als Lagerstabilisator.

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm$^2$ (AUL) durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessig-säure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Üblicherweise wird eine wässrige Monomerlösung verwendet. Der Wassergehalt der Monomerlösung beträgt vorzugsweise von 40 bis 70 Gew.-%, besonders bevorzugt von 45 bis 65 Gew.-%, ganz besonders bevorzugt von 50 bis 60 Gew.-%. Es ist auch möglich Monomersuspensionen, d.h. Monomerlösungen mit der Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Die geschäumte Tensidlösung enthält mindestens ein Tensid. Das mindestens eine Tensid kann ein anionisches, kationisches und/oder nichtionisches Tensid sein. Nichtionische Tenside sind bevorzugt, insbesondere nichtionische Tenside mit einem HLB-Wert von 10 bis 25. Der HLB-Wert ist ein Maß für die Wasser- bzw. Öl-Löslichkeit von vorwiegend nichtionischen Tensiden und kann nach üblichen Methoden bestimmt werden.

Ein Tensid besteht aus mindestens einer unpolaren und mindestens einer polaren Gruppe. Bevorzugte Tenside weisen große unpolare und/oder polare Gruppen auf. Große Gruppen sind Gruppen mit einem Molgewicht von mindestens 130 g/mol, vorzugsweise mindestens 250 g/mol, besonderes bevorzugt mindestens 500 g/mol.

Geeignete Tenside sind beispielsweise Sorbitanester, wie Sorbitanmonostearat, Sorbitanmonooleat, Sorbitanmonopalmitat und Sorbitanmonolaurat, sowie Glycerinester, deren Säurekomponente sich von $C_{14}$- bis $C_{20}$-Carbonsäuren ableitet.

Bevorzugte Tenside sind alkoxilierte, vorzugsweise ethoxilierte, Alkohole, wobei die Alkohole ggf. verzweigt und/oder ungesättigt sein können, sowie alkoxilierte, vorzugsweise ethoxilierte, Sorbitanmonoester, wie Sorbitanmonostearat und Sorbitanmonooleat. Ganz besonders bevorzugte Tenside sind ethoxilierte $C_{14}$-$C_{20}$-Alkohole.

Das mindestens eine Tensid hat vorzugsweise eine Viskosität von über 20 mPas, besonders bevorzugt von über 25 mPas, ganz besonders bevorzugt von über 30 mPas (gemessen bei 23° C gemäß EN12092).

Die geschäumte Tensidlösung kann zusätzlich wasserlösliche Polymere enthalten. Als wasserlösliche Polymere können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Polyglykole, wie Polyethylenglykol, eingesetzt werden.

Die Tensidlösung wird geschäumt. Hierzu sind alle bekannten Methoden zum Aufschäumen geeignet. Die Tensidlösung kann beispielsweise intensiv mit einem inerten Gas, wie Stickstoff oder Kohlendioxid, gemischt werden.

Die geschäumte Tensidlösung wird anschließend mit der Monomerlösung gemischt und polymerisiert. Da der Schaum bereits vorher erzeugt wurde, ist ein intensives Mischen, beispielsweise ein schnelles Rühren, nicht mehr notwendig.

Geeignete Reaktoren für die Polymerisation sind beispielsweise Knetreaktoren oder Bandreaktoren. Im Knetreaktor wird das bei der Polymerisation einer wässrigen Monomerzubereitung entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das zerkleinert werden muss, beispielsweise in einem Extruder oder Knetreaktor.

Zur Verbesserung der Trocknungseigenschaften kann das mittels eines Knetreaktors erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 40 bis 90 mol-%, besonders bevorzugt von 50 bis 85 mol-%, ganz besonders bevorzugt von 60 bis 80 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen. Feste Carbonate und Hydrogencarbonate können hierbei auch in verkapselter Form eingesetzt werden, vorzugsweise in die Monomerlösung direkt vor der Polymerisation, während oder nach der Polymerisation ins Polymergel und vor dessen Trocknung. Die Verkapselung erfolgt durch Beschichtung der Oberfläche mit einem unlöslichen oder nur langsam löslichen Material (beispielsweise mittels filmbildender Polymere, inerter anorganischer Materialien oder schmelzbaren organischer Materialien), welches die Lösung und Reaktion des festen Carbonats oder Hydrogencarbonats so verzögert, dass erst während der Trocknung Kohlendioxid freigesetzt wird und der entstehende Superabsorber eine hohe innere Porosität aufweist.

Das Polymergel wird dann üblicherweise mit einem Umluftbandtrockner getrocknet bis der Restfeuchtegehalt vorzugsweise 0,5 bis 10 Gew.-%, besonders bevorzugt 1 bis 7 Gew.-%, ganz besonders bevorzugt 2 bis 5 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird hiernach üblicherweise gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise von 150

bis 850 $\mu$m, besonders bevorzugt von 250 bis 600 $\mu$m, ganz besonders von 300 bis 500 $\mu$m. Die mittlere Partikelgröße der Produktfraktion kann mittels der von der EDANA empfohlenen Testmethode Nr. WSP 220.2 (05) "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Die Polymerpartikel werden zur weiteren Verbesserung der Eigenschaften thermisch oberflächennachvernetzt. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder $\beta$-Hydroxyalkylamide, wie in DE 102 04 938 A1 und US 6,239,230 beschrieben.

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Hydroxid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumhydroxid, Aluminiumsulfat und Aluminiumlactat sind bevorzugt.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf das Polymer.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächen nachvernetzt.

Bevorzugte Reaktionstemperaturen liegen im Bereich 100 bis 250° C, bevorzugt 110 bis 220° C, besonders bevorzugt 120 bis 210° C, ganz besonders bevorzugt 130 bis 200° C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80° C, besonders bevorzugt bei 35 bis 70° C, ganz besonders bevorzugt bei 40 bis 60° C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Oberflächennachvernetzung durchgeführt.

Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, Fällungskieselsäure, wie Sipernat® D17, und Tenside, wie Span® 20.

Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Herrmann-Debrouxlaan 46, 1160 Oudergem, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von $23 \pm 2\,°$C und einer relativen Luftfeuchte von $50 \pm 10$ % durchgeführt werden. Die wasserabsorbierenden Polymerpartikel werden vor der Messung gut durchmischt.

## Feuchtegehalt

**Der Feuchtegehalt wird gemäß der von der EDANA empfohlenen Testmethode Nr. WSP 230.2 (05) "Mass Loss Upon Heating" bestimmt.**

Zentrifugenretentionskapazität (Centrifuge Retention Capacity)

Die Zentrifugenretentionskapazität (CRC) wird gemäß der von der EDANA empfohlenen Testmethode Nr. WSP 241.2 (05) "Fluid Retention Capacity in Saline, After Centrifugation" bestimmt.

Absorption unter einem Druck von 21,0 $g/cm^2$ (Absorption under Load)

Die Absorption unter einem Druck von 21,0 $g/cm^2$ (AUL) wird gemäß der von der EDANA empfohlenen Testmethode Nr. WSP 242.2 (05) "Absorption Under Pressure, Gravimetric Determination" bestimmt.

Absorption unter einem Druck von 49,2 $g/cm^2$ (Absorption under High Load)

Die Absorption unter einem Druck von 49,2 $g/cm^2$ (AUHL) wird analog der von der EDANA empfohlenen Testmethode Nr. WSP 242.2 (05) "Absorption Under Pressure, Gravimetric Determination" bestimmt, wobei statt eines Drucks von 21,0 $g/cm^2$ (0.3psi) ein Druck von 49,2 $g/cm^2$ (0.7psi) eingestellt wird.

Extrahierbare

Der Gehalt an extrahierbaren Bestandteilen der Superabsorberpartikel wird gemäß der von der EDANA empfohlenen Testmethode Nr. WSP 270.2 (05) "Extractable" bestimmt.

Oberflächenspannung des wässrigen Extraktes

Zur Bestimmung der Oberflächenspannung des wässrigen Extraktes (OFS) werden 0,50 g der Superabsorberpartikel in ein kleines Becherglas eingewogen und mit 40 ml einer 0,9 gew.-%igen Salzlösung versetzt. Der Inhalt des Becherglases wird 3 Minuten bei 500 U/min mit einem Magnetrührstab gerührt, dann lässt man 2 Minuten absitzen. Schließlich wird die Oberflächenspannung der überstehenden wässrigen Phase mit einem Digital-Tensiometer K10-ST oder vergleichbarem Gerät mit Platinplatte gemessen (Krüss GmbH, Hamburg, Deutschland). Die Messung wird bei einer Temperatur von 23°C durchgeführt.

Anquellgeschwindigkeit (Free Swell Rate)

Zur Bestimmung der Anquellgeschwindigkeit (FSR) werden 1,00 g (= W1) der Superabsorberpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Anquellgeschwindigkeit (FSR) wie folgt:

$$\text{FSR [g/gs]} = W2/(W1 \times t)$$

Wenn der Feuchtegehalt der Superabsorberpartikel jedoch mehr als 3 Gew.-% beträgt, so ist das Gewicht W1 um diesen Feuchtegehalt zu korrigieren.

Permeabilität (Saline Flow Conductivity)

Die Permeabilität (SFC) einer gequollenen Gelschicht unter Druckbelastung von 0,3 psi (2070 Pa) wird, wie in EP 2 535 698 A1 beschrieben, mit einer Einwaage von 1,5 g Superabsorberpartikel als Urine Permeability Measurement (UPM) einer gequollenen Gelschicht bestimmt. Der Durchfluss wird automatisch erfasst.

Die Permeabilität (SFC) wird wie folgt berechnet:

$$\text{SFC [cm}^3\text{s/g]} = (Fg(t=0) \times L_0)/(d \times A \times WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, $L_0$ die Dicke der Gelschicht in cm, d die Dichte der NaCl-Lösung in g/cm$^3$, A die Fläche der Gelschicht in cm$^2$ und WP der hydrostatische Druck über der Gelschicht in dyne/cm$^2$.

volumetrische Flüssigkeitsaufnahme unter 0,3 psi (2,07 kPa) Druck (VAUL)

Bei der volumetrischen Flüssigkeitsaufnahme unter 0,3 psi (2,07 kPa) Druck (VAUL) wird der τ-Wert gemäß der in der WO 2014/079694 A1 auf den Seiten 39 und 40 beschriebenen Testmethode "Volumetric Absorbency Under Load (VAUL)" bestimmt. Der τ-Wert wird dort als "characteristic swelling time" bezeichnet.

Beispiele

Herstellung des Grundpolymers

Beispiel 1

Ein Knetreaktor mit zwei Wellen vom Typ LUK 8.0K2 (Coperion Werner & Pfleiderer GmbH & Co. KG, Stuttgart, Deutschland) wurde durch Spülen mit Stickstoff inertisiert. Die Knetreaktorwellen wurden mit 96 Upm bzw. 48 Upm betrieben. Der Mantel des Knetreaktors konnte mittels eines Wärmeträgers beheizt werden.

4901 g einer 37,3 gew.-%igen wässrigen Natriumacrylat -Lösung und 571,9 g Acrylsäure wurden gemischt, durch Spülen mit Stickstoff von Sauerstoff befreit und in den Knetreaktor gefüllt. Anschließend wurden nacheinander eine Mischung aus 7,9 g 3-fach ethoxiliertes Glycerintriacrylat (ca. 85gew.-%ig) und 100 g Acrylsäure, 11,89 g wässrige Natriumperoxodisulfat-Lösung (ca. 15gew.-%ig) und 132 g wässrige Wasserstoffperoxid-Lösung (ca. 3gew.-%ig) in den Knetreaktor dosiert.

Eine Tensidlösung aus 250 g Wasser, 29,6 g wässrigem Polyethylenglykol-4000 (ca. 50gew.-%ig) und 9,9 g 80-fach ethoxiliertem C$_{16}$/C$_{18}$-Fettalkohol (Lutensol® AT80) wurde in einem statischen Mischer mit Stickstoff geschäumt. Polyethylenglykol-4000 ist ein Polyethylenglykol mit einem mittleren Molgewicht von ca. 4.000 g/mol. Der erhaltene stabile Schaum wurde anschließend in den Knetreaktor dosiert. Anschließend wurde die Leitung mit ca. 200 g Wasser gespült.

Anschließend wurde 19,7 g wässrige Ascorbinsäure-Lösung (ca. 0,5gew.-%ig) in den Knetreaktor dosiert und der Mantel des Knetreaktors mittels eines Wärmeträgers (80° C) erwärmt. Die Temperatur im Knetreaktor stieg von 22° C auf 102° C. Sobald der Temperaturanstieg beendet war, wurde die Heizung ausgeschaltet, das erhaltene Polymergel noch 13 Minuten geknetet, auf 63° C gekühlt und aus dem Knetreaktor ausgetragen.

Das erhaltene Polymergel wurde in Portionen zu ca. 1.080 g homogen auf Drahtnetzböden verteilt und in einem Umlufttrockenschrank für 90 Minuten bei 175° C getrocknet. Das erhaltene getrocknete Polymergel wurde mit einem Walzenstuhl vom Typ LRC 250 (Bauermeister Zerkleinerungstechnik GmbH, Norderstedt, Deutschland) dreistufig gemahlen (1.000 $\mu$m, 600 $\mu$m und 400 $\mu$m) und auf eine Partikelgröße von 150 bis 710 $\mu$m abgesiebt.

Die erhaltenen Superabsorberpartikel wurden analysiert. Die Ergebnisse sind in Tabelle 1 zusammengefasst.

Beispiel 2

Es wurde verfahren wie in Beispiel 1, wobei als Tensidlösung eine Lösung aus 250 g Wasser, 14,7 g wässrigem Polyethylenglykol-4000 (ca. 50gew.-%ig) und 4,9 g 80-fach ethoxiliertem $C_{16}$/$C_{18}$-Fettalkohol (Lutensol® AT80) verwendet wurde.

Die erhaltenen Superabsorberpartikel wurden analysiert. Die Ergebnisse sind in Tabelle 1 zusammengefasst.

Beispiel 3 (Vergleichsbeispiel)

Es wurde verfahren wie in Beispiel 1, wobei nicht geschäumt wurde.

Die erhaltenen Superabsorberpartikel wurden analysiert. Die Ergebnisse sind in Tabelle 1 zusammengefasst.

Beispiel 4 (Vergleichsbeispiel)

Es wurde verfahren wie in Beispiel 1, wobei statt der Tensidlösung wurde eine Lösung aus 250 g Wasser und 29,6 g wässrigem Polyethylenglykol-4000 (ca. 50gew.-%ig) verwendet und nicht geschäumt wurde.

Die erhaltenen Superabsorberpartikel wurden analysiert. Die Ergebnisse sind in Tabelle 1 zusammengefasst.

Beispiel 5 (Vergleichsbeispiel)

Es wurde verfahren wie in Beispiel 1, wobei statt der Tensidlösung wurde eine Tensidlösung aus 250 g Wasser und 9,9 g 80-fach ethoxiliertem $C_{16}$/$C_{18}$-Fettalkohol (Lutensol® AT80) verwendet und nicht geschäumt wurde.

Die erhaltenen Superabsorberpartikel wurden analysiert. Die Ergebnisse sind in Tabelle 1 zusammengefasst.

Beispiel 6 (Vergleichsbeispiel)

Es wurde verfahren wie in Beispiel 1, wobei die Tensidlösung zu der Monomerlösung in den Knetreaktor dosiert wurde und erst die Mischung aus Monomerlösung und Tensidlösung im Knetreaktor mit einem zylindrischen gesinterten Filterelement vom Typ SIK-R 15 AX (GKN Sinter Metal Filters GmbH, Radevormwald, Deutschland) für zwei Minuten mit Stickstoff (1 bar, 1166 ml/min) geschäumt wurde.

Die erhaltenen Superabsorberpartikel wurden analysiert. Die Ergebnisse sind in Tabelle 1 zusammengefasst.

Tab. 1: Eigenschaften der Grundpolymere

| Bsp. | CRC [g/g] | AUL [g/g] | FSR [g/gs] | Ext. [Gew.-%] | OFS [mN/m] | $T_{max}$ [° C] |
|------|-----------|-----------|------------|---------------|------------|------------------|
| 1 | 38,4 | 11,9 | 0,33 | 14,5 | 56,7 | 102 |
| 2 | 37,4 | 15,2 | 0,31 | 11,4 | 55,1 | 95 |
| 3*) | 35,9 | 18,7 | 0,31 | 7,9 | 54,1 | 89 |
| 4*) | 35,9 | 20,5 | 0,31 | 8,7 | 63,7 | 91 |
| 5*) | 34,5 | 23,2 | 0,34 | 8,6 | 54,7 | 93 |
| 6*) | 35,3 | 22,8 | 0,33 | 8,2 | 53,8 | 88 |
| *) Vergleichsbeispiel | | | | | | |

Oberflächennachvernetzung

Beispiel 7

1.200 g Grundpolymer aus Beispiel 1 wurde in einem Pflugschar-Mischer vom Typ M5 mit Heizmantel (Gebr. Lödige Maschinenbau GmbH; Paderborn, Deutschland) bei 23° C und einer Wellendrehzahl von 200 Umdrehungen pro Minute mittels einer Zweistoff-Sprühdüse mit 54,4 g eines Gemisches aus 1,5 Gew.-% N-Hydroxyethyl-2-oxazolidinon, 1,5 Gew.-% 1,3-Propandiol, 26,7 Gew.-% Isopropanol, 11,0 Gew.-% Aluminiumlaktat und 59,3 Gew.-% Wasser beschichtet.

Nach dem Aufsprühen wurde die Wellendrehzahl auf 50 Umdrehungen pro Minute reduziert und die Produkttemperatur auf 185° C erhöht. Anschließend wurde das Reaktionsgemisch 40 Minuten lang bei dieser Temperatur und dieser Wellendrehzahl gehalten. Das erhaltene Produkt wurde auf Umgebungstemperatur abgekühlt und erneut mit einem 710$\mu$m Sieb klassiert.

Die erhaltenen Superabsorberpartikel wurden analysiert. Die Ergebnisse sind in Tabelle 2 zusammengefasst.

Beispiel 8

Es wurde verfahren wie in Beispiel 7, wobei das Grundpolymer aus Beispiel 2 eingesetzt wurde
Die erhaltenen Superabsorberpartikel wurden analysiert. Die Ergebnisse sind in Tabelle 2 zusammengefasst.

Beispiel 9 (Vergleichsbeispiel)

Es wurde verfahren wie in Beispiel **7,** wobei das Grundpolymer aus Beispiel 3 eingesetzt wurde
Die erhaltenen Superabsorberpartikel wurden analysiert. Die Ergebnisse sind in Tabelle 2 zusammengefasst.

Beispiel 10 (Vergleichsbeispiel)

Es wurde verfahren wie in Beispiel **7,** wobei das Grundpolymer aus Beispiel 4 eingesetzt wurde
Die erhaltenen Superabsorberpartikel wurden analysiert. Die Ergebnisse sind in Tabelle 2 zusammengefasst.

Beispiel 11 (Vergleichsbeispiel)

Es wurde verfahren wie in Beispiel 7, wobei das Grundpolymer aus Beispiel 5 eingesetzt wurde
Die erhaltenen Superabsorberpartikel wurden analysiert. Die Ergebnisse sind in Tabelle 2 zusammengefasst.

Beispiel 12 (Vergleichsbeispiel)

Es wurde verfahren wie in Beispiel 7, wobei das Grundpolymer aus Beispiel 6 eingesetzt wurde
Die erhaltenen Superabsorberpartikel wurden analysiert. Die Ergebnisse sind in Tabelle 2 zusammengefasst.

Tab. 2: Eigenschaften nach der Oberflächennachvernetzung

| Bsp. | SFC [$10^{-7}$ cm$^3$s/g] | CRC [g/g] | AUHL [g/g] | VAUL [s] |
|------|---------------------------|-----------|------------|----------|
| 7 | 35 | 30,0 | 27,4 | 162 |
| 8 | 33 | 31,0 | 27,6 | 196 |
| 9*) | 41 | 29,6 | 26,0 | 240 |
| 10*) | 39 | 29,1 | 26,2 | 232 |
| 11*) | 49 | 28,4 | 26,3 | 221 |
| 12*) | 47 | 30,0 | 26,5 | 254 |
| *) Vergleichsbeispiel | | | | |

Der Vergleich der Beispiele 7 und 12 zeigt, dass das Schäumen der Tensidlösung in Abwesenheit der Monomerlösung (Beispiel 7) zu einem deutlich anderen Eigenschaftsprofil führt als das Schäumen in Gegenwart der Monomerlösung (Beispiel 12).

**Patentansprüche**

1.  Verfahren zur Herstellung von Superabsorberpartikeln, umfassend Polymerisation einer Monomerzubereitung, Trocknung des erhaltenen wässrigen Polymergels, Mahlung, Klassierung und thermische Oberflächennachvernetzung, **dadurch gekennzeichnet, dass** die Monomerzubereitung durch Mischen einer wässrigen Monomerlösung, enthaltend

    a) mindestens ein ethylenisch ungesättigtes, säuregruppentragendes Monomer, das zumindest teilweise neutralisiert ist,
    b) mindestens einen Vernetzer und
    c) mindestens einen Initiator,

    und einer geschäumten wässrigen Tensidlösung hergestellt wird.

2.  Verfahren nach Anspruch 1, **dadurch gekennzeichnet, dass** die Polymerisation der Monomerzubereitung in einem Knetreaktor durchgeführt wird und die wässrige Monomerlösung und die geschäumte wässrige Tensidlösung im Knetreaktor gemischt werden.

3.  Verfahren nach Anspruch 1 oder 2, **dadurch gekennzeichnet, dass** die Polymerisation in einem kontinuierlichen Knetreaktor durchgeführt wird.

4.  Verfahren nach einem der Ansprüche 1 bis 3, **dadurch gekennzeichnet, dass** die wässrige Monomerlösung von 40 bis 50 Gew.-% des Monomeren a) enthält.

5.  Verfahren nach einem der Ansprüche 1 bis 4, **dadurch gekennzeichnet, dass** das Monomer a) zu 60 bis 80 mol-% neutralisiert ist.

6.  Verfahren nach einem der Ansprüche 1 bis 5, **dadurch gekennzeichnet, dass** das Monomer a) teilweise neutralisierte Acrylsäure ist

7.  Verfahren nach einem der Ansprüche 1 bis 6, **dadurch gekennzeichnet, dass** die geschäumte wässrige Tensidlösung von 1 bis 3 Gew.-% mindestens eines Tensids enthält.

8.  Verfahren nach einem der Ansprüche 1 bis 7, **dadurch gekennzeichnet, dass** die geschäumte wässrige Tensidlösung mindestens ein nichtionisches Tensid enthält.

9.  Verfahren nach einem der Ansprüche 1 bis 8, **dadurch gekennzeichnet, dass** das Gewichtsverhältnis von geschäumter wässriger Tensidlösung zu wässriger Monomerlösung in der Monomerzubereitung von 0,03 bis 0,10 beträgt.

10. Verfahren nach einem der Ansprüche 1 bis 9, **dadurch gekennzeichnet, dass** die geschäumte wässrige Tensidlösung zusätzlich ein wasserlösliches Polymer enthält.

11. Verfahren nach Anspruch 10, **dadurch gekennzeichnet, dass** die geschäumte wässrige Tensidlösung von 5 bis 10 Gew.-% des zusätzlichen wasserlöslichen Polymers enthält.

12. Verfahren nach Anspruch 10 oder 11, **dadurch gekennzeichnet, dass** das zusätzliche wasserlösliche Polymer Polyethylenglykol ist.

13. Verfahren nach einem der Ansprüche 1 bis 12, **dadurch gekennzeichnet, dass** der mindestens eine Initiator c) ein Redox-Initiator ist.

**Claims**

1.  A process for producing superabsorbent particles, comprising polymerization of a monomer preparation, drying of the resultant aqueous polymer gel, grinding, classifying and thermal surface postcrosslinking, which comprises producing the monomer preparation by mixing an aqueous monomer solution comprising

    a) at least one ethylenically unsaturated monomer which bears acid groups and is at least partly neutralized,
    b) at least one crosslinker and
    c) at least one initiator,

    and a foamed aqueous surfactant solution.

**2.** The process according to claim 1, wherein the polymerization of the monomer preparation is conducted in a kneading reactor and the aqueous monomer solution and the foamed aqueous surfactant solution are mixed in the kneading reactor.

**3.** The process according to claim 1 or 2, wherein the polymerization is conducted in a continuous kneading reactor.

**4.** The process according to any of claims 1 to 3, wherein the aqueous monomer solution comprises from 40% to 50% by weight of monomer a).

**5.** The process according to any of claims 1 to 4, wherein monomer a) has been neutralized to an extent of 60 to 80 mol%.

**6.** The process according to any of claims 1 to 5, wherein monomer a) is partly neutralized acrylic acid.

**7.** The process according to any of claims 1 to 6, wherein the foamed aqueous surfactant solution comprises from 1% to 3% by weight of at least one surfactant.

**8.** The process according to any of claims 1 to 7, wherein the foamed aqueous surfactant solution comprises at least one nonionic surfactant.

**9.** The process according to any of claims 1 to 8, wherein the weight ratio of foamed aqueous surfactant solution to aqueous monomer solution in the monomer preparation is from 0.03 to 0.10.

**10.** The process according to any of claims 1 to 9, wherein the foamed aqueous surfactant solution additionally comprises a water-soluble polymer.

**11.** The process according to claim 10, wherein the foamed aqueous surfactant solution comprises from 5% to 10% by weight of the additional water-soluble polymer.

**12.** The process according to claim 10 or 11, wherein the additional water-soluble polymer is polyethylene glycol.

**13.** The process according to any of claims 1 to 12, wherein the at least one initiator c) is a redox initiator.

**Revendications**

**1.** Procédé de production de particules superabsorbantes, comprenant la polymérisation d'une préparation de monomères, le séchage du gel polymère aqueux obtenu, le broyage, la classification et la post-réticulation thermique de la surface, **caractérisé en ce que** la préparation de monomères est préparée par mélange d'une solution aqueuse de monomères, contenant

    a) au moins un monomère éthyléniquement insaturé, portant des groupes acides, qui est au moins partiellement neutralisé,
    b) au moins un réticulant et
    c) au moins un initiateur,

    et d'une solution aqueuse moussée de tensioactif.

**2.** Procédé selon la revendication 1, **caractérisé en ce que** la polymérisation de la préparation de monomères est effectuée dans un réacteur de malaxage et la solution aqueuse de monomères et la solution aqueuse moussée de tensioactif sont mélangées dans le réacteur de malaxage.

**3.** Procédé selon la revendication 1 ou 2, **caractérisé en ce que** la polymérisation est réalisée dans un réacteur de malaxage continu.

4. Procédé selon l'une quelconque des revendications 1 à 3, **caractérisé en ce que** la solution aqueuse de monomères contient de 40 à 50 % en poids du monomère a).

5. Procédé selon l'une quelconque des revendications 1 à 4, **caractérisé en ce que** le monomère a) est neutralisé à 60 à 80 % en moles.

6. Procédé selon l'une quelconque des revendications 1 à 5, **caractérisé en ce que** le monomère a) est l'acide acrylique partiellement neutralisé.

7. Procédé selon l'une quelconque des revendications 1 à 6, **caractérisé en ce que** la solution aqueuse moussée de tensioactif de 1 à 3 % en poids contient au moins un tensioactif.

8. Procédé selon l'une quelconque des revendications 1 à 7, **caractérisé en ce que** la solution aqueuse moussée de tensioactif contient au moins un tensioactif non ionique.

9. Procédé selon l'une quelconque des revendications 1 à 8, **caractérisé en ce que** le rapport pondéral de la solution aqueuse moussée de tensioactif à la solution aqueuse de monomères dans la préparation de monomères est de 0,03 à 0,10.

10. Procédé selon l'une quelconque des revendications 1 à 9, **caractérisé en ce que** la solution aqueuse moussée de tensioactif contient en outre un polymère hydrosoluble.

11. Procédé selon la revendication 10, **caractérisé en ce que** la solution aqueuse moussée de tensioactif contient de 5 à 10 % en poids du polymère hydrosoluble supplémentaire.

12. Procédé selon la revendication 10 ou 11, **caractérisé en ce que** le polymère hydrosoluble supplémentaire est le polyéthylèneglycol.

13. Procédé selon l'une quelconque des revendications 1 à 12, **caractérisé en ce que** l'au moins un initiateur c) est un initiateur redox.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2518092 A1
- DE 102005011165 A1
- EP 0530438 A1
- EP 0547847 A1
- EP 0559476 A1
- EP 0632068 A1
- WO 9321237 A1
- WO 03104299 A1
- WO 03104300 A1
- WO 03104301 A1
- DE 10331450 A1
- DE 10331456 A1
- DE 10355401 A1
- DE 19543368 A1
- DE 19646484 A1

- WO 9015830 A1
- WO 02032962 A2
- WO 2001038402 A1
- DE 3825366 A1
- US 6241928 B
- EP 0083022 A2
- EP 0543303 A1
- EP 0937736 A2
- DE 3314019 A1
- DE 3523617 A1
- EP 0450922 A2
- DE 10204938 A1
- US 6239230 B
- EP 2535698 A1
- WO 2014079694 A1

**In der Beschreibung aufgeführte Nicht-Patentliteratur**

- **F.L. BUCHHOLZ** ; **A.T. GRAHAM**. Modern Super-absorbent Polymer Technology. Wiley-VCH, 1998, 71-103